# EUROPÄISCHE PATENTSCHRIFT

(11) **EP 3 288 605 B1**
(45) Veröffentlichungstag und Bekanntmachung des Hinweises auf die Patenterteilung: **30.01.2019**
(21) Anmeldenummer: 16716843.4
(22) Anmeldetag: 07.04.2016
(51) Int. Cl.: A61M 1/00, A61F 9/007, A61B 17/00

(54) **STEUERUNGSVORRICHTUNG FÜR EIN PHAKOEMULSIFIKATIONSSYSTEM UND PHAKOEMULSIFIKATIONSSYSTEM MIT EINER SOLCHEN STEUERUNGSVORRICHTUNG**
CONTROL DEVICE FOR A PHACOEMULSIFICATION SYSTEM AND PHACOEMULSIFICATION SYSTEM COMPRISING SUCH A CONTROL DEVICE
DISPOSITIF DE COMMANDE POUR UN SYSTÈME DE PHACOÉMULSIFICATION ET SYSTÈME DE PHACOÉMULSIFICATION DOTÉ D'UN TEL DISPOSIITF DE COMMANDE

(30) Priorität: 25.04.2015 DE 102015005331
(43) Veröffentlichungstag der Anmeldung: 07.03.2018
(73) Patentinhaber: Carl Zeiss Meditec AG, 07745 Jena (DE)
(72) Erfinder: KOHLHAMMER, Susanne, 89134 Blaustein (DE); FANENBRUCK, Martin, 73447 Oberkochen (DE); LANGHEINRICH, Peter, 73447 Oberkochen (DE)
(74) Vertreter: Carl Zeiss AG - Patentabteilung
(86) Internationale Anmeldenummer: PCT/EP2016/057561
(87) Internationale Veröffentlichungsnummer: WO 2016/173816

(56) Entgegenhaltungen:
- EP-B1- 1 765 190
- DE-A1-102008 046 687
- DE-A1-102012 018 982
- DE-B3-102011 114 584

## Beschreibung

Die Erfindung betrifft eine Steuerungsvorrichtung für ein Phakoemulsifikationssystem und ein Phakoemulsifikationssystem mit einer solchen Steuerungsvorrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Hohlnadel (Phakonadel) in die Augenlinse eingeführt und mit Ultraschall zu Schwingungen angeregt wird. Die vibrierende Hohlnadel emulsifiziert in ihrer nächsten Umgebung die Augenlinse derart, dass die entstehenden Linsenpartikel durch die Hohlnadel und eine daran angeschlossene Leitung, welche zusammen eine Aspirationsleitung bilden, mittels einer Pumpe abgesaugt werden können. Dabei wird ein Spülfluid (Irrigationsfluid) zugeführt. Ist die Augenlinse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Um in möglichst kurzer Zeit eine vollständige Emulsifikation der Augenlinse und somit für einen Patienten eine möglichst kurze Operationsdauer zu erreichen, ist es sinnvoll, die Hohlnadel mit möglichst großen Longitudinal-Amplituden zu bewegen. Dies lässt sich derart durchführen, dass einem Aktuator in Form von piezoelektrischen Elementen elektrische Energie so zugeführt wird, dass der Aktuator mit der daran gekoppelten Hohlnadel eine Schwingung im Bereich der Resonanzfrequenz durchführt.

Erfolgt für einen solchen Betrieb eine Zufuhr von elektrischer Energie an die piezoelektrischen Elemente während einer genügend langen Zeit, erwärmen sich nicht nur die piezokeramischen Elemente. Auch der Umgebungsbereich der mit den piezoelektrischen Elementen gekoppelten Hohlnadel erwärmt sich so sehr, dass die für die Phakoemulsifikation durchstochene Hornhaut in der Umgebung der Hohlnadel verbrennen kann. Eine solche Verletzung sollte unbedingt vermieden werden. Insofern besteht ein Konflikt darin, möglichst viel Energie zur Emulsifikation einer Augenlinse zuzuführen, dabei jedoch keine zu starke Erwärmung der Hornhaut zu verursachen.

In der US 2007/ 0 078 379 A1 sind eine Vielzahl an Pulsen vorgeschlagen, mit welchen die Phakonadel bewegt werden kann. Die Pulse weisen lineare Anstiege von einer niedrigen Amplitude zu einer höheren Amplitude und lineare Abstiege von der höheren Amplitude zu der niedrigeren Amplitude auf. Dies soll eine Verbesserung zu den bisher bekannten Rechteckpulsen darstellen, da bei Rechteckpulsen Linsenpartikel an der Phakonadel nicht gut gehalten und sogar von der Phakonadel fortgestoßen würden, sodass nur eine sehr schlechte Emulsifizierung möglich wäre. Damit trotzdem eine ausreichende Emulsifizierung erfolgen könnte, müsste an die mit Rechteckpulsen bewegte Phakonadel eine sehr hohe Energie zugeführt werden. Dies bedeutet nichts anderes, als dass die Rechteckpulse eine sehr hohe Amplitude aufweisen müssten, wodurch aber eine exzessive Wärme in das Gewebe eingebracht würde, was es zu verhindern gelte. Außerdem würden durch Rechteckpulse Kavitationseffekte, also implodierende Blasen, erzeugt, wodurch ebenfalls unerwünschte Wärme und unerwünschte zusätzliche Kräfte in das Gewebe eingebracht würden.

In der US 2010/0268388 A1 ist ein Verfahren zur Steuerung eines chirurgischen Systems basierend auf einem Irrigationsvolumenstrom beschrieben. Dazu wird eine Temperatur des Auges während der Operation bestimmt, wobei die der Phakonadel zugeführte Energie reduziert wird, wenn aufgrund der bestimmten Temperatur eine Überhitzung des Gewebes zu erwarten ist. Die Energie wird derart reduziert, dass die Höhe der Rechteckpulse, also ihre Amplitude, reduziert wird.

Es besteht eine Aufgabe darin, eine Steuerungsvorrichtung für ein Phakoemulsifikationssystem und ein Phakoemulsifikationssystem mit einer solchen Steuerungsvorrichtung zu schaffen, mit der bei geringem Steuerungsaufwand die Emulsifikation einer Augenlinse in kurzer Zeit mit hohem Wirkungsgrad und geringer Gefahr für eine Verbrennung der Hornhaut erreicht werden kann.

Die Aufgabe wird für die Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe wird ferner durch ein Phakoemulsifikationssystem gemäß dem unabhängigen Anspruch 8 gelöst.

Die erfindungsgemäße Steuerungsvorrichtung für ein Phakoemulsifikationssystem ist eingerichtet, einem Aktuator für eine Phakonadel während mehrerer Zeitabschnitte elektrische Energie zuzuführen, wobei die Zeitabschnitte umfassen:
- einen ersten Zeitabschnitt, in dem elektrische Energie für Pulse für eine Phakoemulsifikation zugeführt wird, wobei die Pulse eine konstante maximale Amplitude aufweisen,
- einen zweiten Zeitabschnitt, welcher sich dem ersten Zeitabschnitt anschließt, wobei in dem zweiten Zeitabschnitt elektrische Energie mit einem Betrag gleich Null zugeführt wird,
- einen dritten Zeitabschnitt, welcher sich dem zweiten Zeitabschnitt anschließt, wobei der dritte Zeitabschnitt eine erste Zeitdauer aufweist, in der elektrische Energie für Pulse für eine Phakoemulsifikation zugeführt wird, wobei die Pulse eine niedrigere konstante Amplitude als die maximale Amplitude während des ersten Zeitabschnittes aufweisen,
wobei auf die erste Zeitdauer eine zweite Zeitdauer folgt, in der Pulse mit einer Amplitude anliegen, die einen konstanten Betrag in Höhe von mehr als 0 % und weniger als 10 % der maximalen Amplitude erreicht, sodass während der zweiten Zeitdauer keine Phakoemulsifikation erfolgt,
wobei die erste Zeitdauer des dritten Zeitabschnittes kürzer ist als der erste Zeitabschnitt.

Die erfindungsgemäße Steuerungsvorrichtung ist somit derart eingerichtet, dass während des ersten Zeitabschnittes mit einer maximalen Amplitude relativ viel Energie in die Augenlinse eingebracht wird, um eine wirksame Emulsifikation der Augenlinse beginnen zu können. Danach folgt mit dem zweiten Zeitabschnitt eine Pause, in der keine Energie zugeführt wird, sodass eine Abkühlung der Phakonadel und der diese umgebenden Hornhaut eintreten kann. Diese Abkühlung wird jedoch nicht so lange zugelassen, dass eine Temperatur wie zu Beginn des ersten Zeitabschnittes vorliegt, da dies relativ viel Zeit erfordern würde und die Phakoemulsifikation nicht schnell genug durchgeführt werden könnte. Daher ist die Steuerungsvorrichtung eingerichtet, dass nach Ablauf des zweiten Zeitabschnittes ein dritter Zeitabschnitt mit einer ersten Zeitdauer folgt, in der elektrische Energie an den Aktuator für Pulse zugeführt wird, welche eine niedrigere Amplitude als während des ersten Zeitabschnittes aufweisen. Die niedrigere Amplitude bewirkt, dass weniger Energie als im ersten Zeitabschnitt zugeführt wird, sodass zwar eine Phakoemulsifikation erfolgt, jedoch mit geringerer Intensität. Eine Abkühlung kann während der ersten Zeitdauer weiterhin erfolgen. Die während der sich an die erste Zeitdauer anschließenden zweiten Zeitdauer zugeführte Energie ist so niedrig, dass keine Phakoemulsifikation mehr erfolgt, wodurch eine Abkühlung immer noch fortgesetzt werden kann. Jedoch hat die während der zweiten Zeitdauer im dritten Zeitabschnitt zugeführte Energie nicht den Betrag Null, sodass eine Schwingung des Aktuators weiterhin erfolgt. Dies ist vorteilhaft, da während des gesamten dritten Zeitabschnitts das Schwingungsverhalten des Aktuators in Form von piezoelektrischen Elementen ausgewertet werden kann. Zum Beispiel kann die Lage der Resonanzfrequenz oder ein Okklusionszustand an der Phakonadel überprüft werden. Wenn sich an den dritten Zeitabschnitt eine Wiederholung des ersten bis dritten Zeitabschnittes anschließt, kann die Schwingung des Aktuators wieder in einer neu eingestellten Resonanzfrequenz erfolgen.

Die Erfinder haben festgestellt, dass es durch Einsatz der erfindungsgemäßen Steuerungsvorrichtung möglich ist, eine wirksame Emulsifikation der Augenlinse in kurzer Zeit durchzuführen, während gleichzeitig eine geringe thermische Belastung des die Phakonadel umgebenden Gewebes und insbesondere der Hornhaut auftritt. Die Erfinder haben zusätzlich die besonders vorteilhafte Eigenschaft festgestellt, dass sich durch Einsatz der erfindungsgemäßen Steuerungsvorrichtung aufgrund der erwähnten Zeitabschnitte die erzeugten Linsenpartikel gut in die Aspirationsleitung absaugen lassen, obwohl diese durch die gleichzeitig in Longitudinalrichtung schwingende Hohlnadel von der Nadelspitze fortgestoßen werden. Von Bedeutung ist dabei besonders der zweite Zeitabschnitt, in welchem dem Aktuator der Phakonadel keine Energie zugeführt wird. Ferner haben die Erfinder beobachtet, dass durch Einsatz der erfindungsgemäßen Steuerungsvorrichtung aufgrund der erwähnten Zeitabschnitte bei einer relativ harten Augenlinse ein Kontakt der longitudinal schwingenden Spitze der Hohlnadel zu der Augenlinse immer noch relativ gut ist, so dass eine gute Emulsifikation erreicht wird. Durch Einsatz der erfindungsgemäßen Steuerungsvorrichtung werden die Linsenpartikel somit gut an der Nadel gehalten, wobei insgesamt eine Emulsifikation der Augenlinse mit hohem Wirkungsgrad bei geringer Gefahr einer Verbrennung der Hornhaut möglich ist.

Gemäß einer Ausführungsform der Erfindung ist die Länge des ersten Zeitabschnittes vorbestimmt, wobei durch eine Verstellung eines Fußpedals nur die Länge des dritten Zeitabschnittes veränderbar ist. Dadurch wird im ersten Zeitabschnitt immer genügend Energie der Augenlinse zugeführt, wobei die Zeit für die Abkühlung im dritten Zeitabschnitt an die jeweilige Situation während einer ophthalmochirurgischen Operation angepasst werden kann. Der Operateur entscheidet aufgrund seiner Erfahrung und Beobachtung während der Operation, wie er das Fußpedal betätigt, um die Zeit für den dritten Zeitabschnitt entsprechend einzustellen. Es wird also keine Steuerung aufgrund einer gemessenen Temperatur an der Phakonadel vorgenommen. Es erfolgt auch keine Steuerung aufgrund einer Belastung der Phakonadel mit einem Linsenpartikel während der Operation. Es erfolgt auch keine Bildverarbeitung von der Umgebung des distalen Endes der Phakonadel. Und es erfolgt auch keine automatische Regelung des Pulsmodi aufgrund einer Änderung eines chirurgischen Parameters. Auf derartige Ansätze wird bei der erfindungsgemäßen Steuerungsvorrichtung bewusst verzichtet. Es erfolgt zum Beispiel an einer grafischen Bedienoberfläche nur die Voreinstellung einer Länge des ersten Zeitabschnittes und vorzugsweise auch einer Länge des zweiten Zeitabschnittes, wobei nur eine Veränderung des Betrages für den dritten Zeitabschnitt mittels eines Fußpedals durch den Operateur erfolgt.

Gemäß einer weiteren Ausführungsform der Erfindung ist die Länge des dritten Zeitabschnittes und auch vorzugsweise des zweiten Zeitabschnittes vorbestimmt, zum Beispiel durch Einstellung an einer grafischen Bedienoberfläche, wobei durch eine Verstellung eines Fußpedals nur die Länge des ersten Zeitabschnittes veränderbar ist. Damit kann der Operateur einen insgesamt relativ geringen Energieeintrag in die Augenlinse erreichen.

Vorzugsweise ist die Amplitude der Pulse der ersten Zeitdauer des dritten Zeitabschnittes in einem Bereich von 40 % bis 70 % der maximalen Amplitude des ersten Zeitabschnittes einstellbar. Damit wird erreicht, dass eine Phakoemulsifikation noch stattfindet, jedoch nur mit einem niedrigeren Energiebetrag, um ein weiteres starkes Aufheizen der Umgebung der Phakonadel zu vermeiden.

Gemäß einer Weiterbildung der Erfindung weist der dritte Zeitabschnitt eine erste Zeitdauer und eine zweite Zeitdauer und direkt daran anschließend eine dritte Zeitdauer und eine vierte Zeitdauer auf, wobei in der dritten Zeitdauer elektrische Energie für Pulse zugeführt wird, welche eine niedrigere konstante Amplitude als während des ersten Zeitabschnittes aufweisen und in der vierten Zeitdauer Pulse mit einer Amplitude anliegen, die einen konstanten Betrag in Höhe von mehr als 0 % und weniger als 10 % der maximalen Amplitude erreicht. Vorzugsweise ist die Amplitude der Pulse der dritten Zeitdauer des dritten Zeitabschnittes in einem Bereich von 40 % bis 70 % der maximalen Amplitude des ersten Zeitabschnittes einstellbar. Damit wird eine weitere Abkühlung im dritten Zeitabschnitt ermöglicht, während trotzdem noch in einem ausreichenden Maß eine Emulsifikation möglich ist. Zusätzlich kann während des gesamten dritten Zeitabschnittes das Schwingungsverhalten des Aktuators ausgewertet werden.

Vorzugsweise ist durch eine Verstellung des Fußpedals das Verhältnis aus dem dritten Zeitabschnitt zu dem ersten Zeitabschnitt in einem Bereich von 0,5 bis 2, bevorzugt in einem Bereich von 0,5 bis 1, verstellbar. Der dritte Zeitabschnitt ist somit mindestens halb so lang und höchstens doppelt so lang wie der erste Zeitabschnitt.

Gemäß einer weiteren Ausführungsform der Erfindung weisen die Pulse bei der dritten Zeitdauer eine niedrigere konstante Amplitude als bei der ersten Zeitdauer auf. Bevorzugt liegt die Amplitude in der dritten Zeitdauer in einem Bereich von 15 % bis 35 % der maximalen Amplitude des ersten Zeitabschnittes. Damit ist eine noch geringere Energiezufuhr während der dritten Zeitdauer sichergestellt, sodass eine noch zuverlässigere Abkühlung der Umgebung der Phakonadel möglich ist.

Weitere Vorteile und Merkmale der Erfindung werden mit Bezug auf die nachfolgenden Zeichnungen erklärt, in welchen zeigen:
- Figur 1: eine schematische Darstellung eines erfindungsgemäßen Phakoemulsifikationssystems ;
- Figur 2: eine schematische Darstellung eines Pulsverlaufes in Abhängigkeit von der Zeit gemäß einer ersten Ausführungsform der erfindungsgemäßen Steuerungsvorrichtung;
- Figur 3: eine schematische Darstellung eines Pulsverlaufes in Abhängigkeit von der Zeit gemäß einer zweiten Ausführungsform der erfindungsgemäßen Steuerungsvorrichtung; und
- Figur 4: eine schematische Darstellung eines Pulsverlaufes in Abhängigkeit von der Zeit gemäß einer dritten Ausführungsform der erfindungsgemäßen Steuerungsvorrichtung.

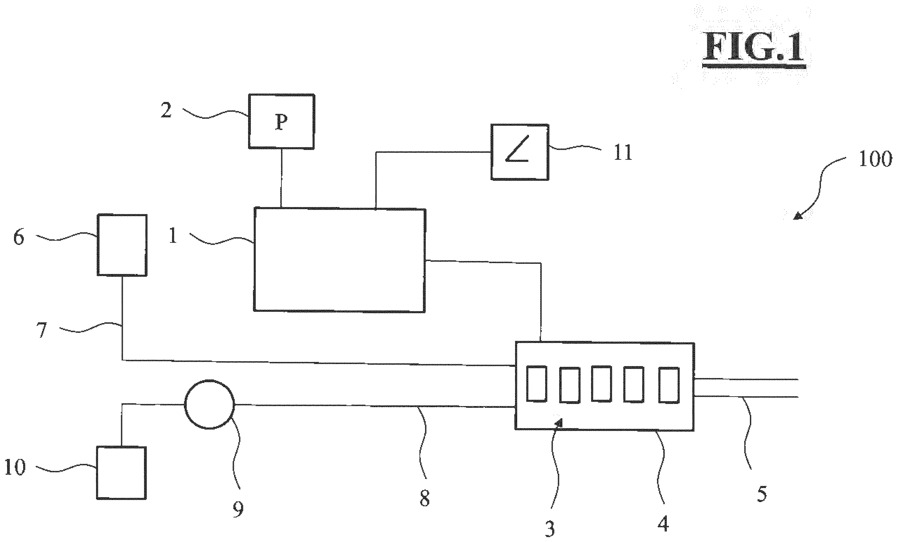

Figur 1 zeigt eine schematische Darstellung eines erfindungsgemäßen Phakoemulsifikationssystems 100. Das Phakoemulsifikationssystem 100 weist eine Steuerungsvorrichtung 1 auf, welche mit einer Energieversorgung 2 gekoppelt ist. Die Energieversorgung 2 ist erforderlich, um einen Aktuator 3 aus zum Beispiel piezoelektrischen Elementen in einem Phakohandstück 4 mit Energie zu versorgen, sodass eine mit dem Aktuator 3 gekoppelte Phakonadel 5 eine Longitudinal-Schwingung ausführen kann. Im Bereich eines distalen Endes der Phakonadel 5 wird Irrigationsfluid 6 mittels einer Irrigationsleitung 7 zugeführt, welches zusammen mit emulsifizierten Linsenpartikeln mittels einer Aspirationsleitung 8 von einer Aspirationspumpe 9 zu einem Aspirationsfluidbehälter 10 transportiert wird. Die Steuerungsvorrichtung 1 ist ferner mit einem Fußpedal 11 gekoppelt, sodass sich in Abhängigkeit von der Stellung des Fußpedals 11 der Aktuator 3 des Phakohandstückes von der Steuerungsvorrichtung 1 ansteuern lässt.

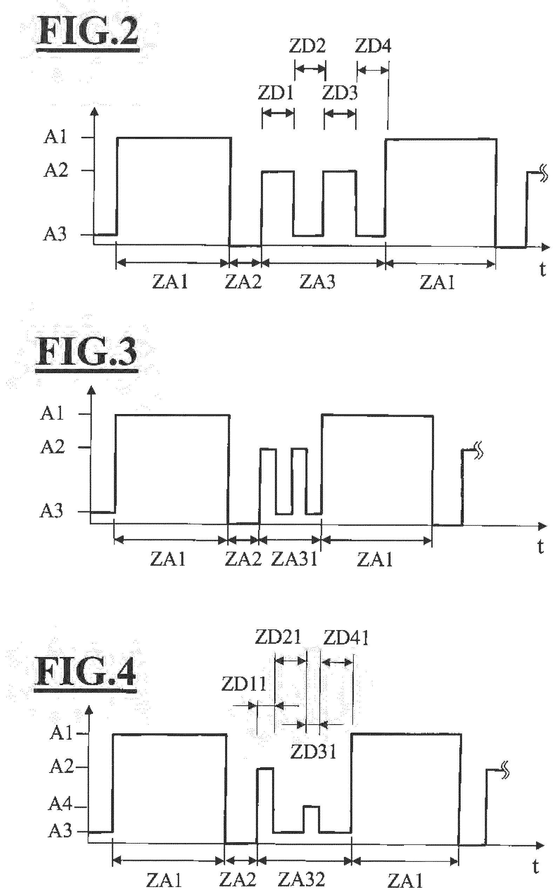

Figur 2 zeigt eine schematische Darstellung eines Pulsverlaufes in Abhängigkeit von der Zeit gemäß einer ersten Ausführungsform der Steuerungsvorrichtung 1. Während eines ersten Zeitabschnittes ZA1 wird dem Aktuator 3 des Handstückes 4 elektrische Energie zugeführt, sodass die Phakonadel 5 in eine Schwingung versetzt wird, welche eine konstante maximale Amplitude A1 aufweist. Nach Ablauf des ersten Zeitabschnittes ZA1 folgt ein zweiter Zeitabschnitt ZA2, in welchem dem Aktuator 3 von der Steuerungsvorrichtung 1 eine elektrische Energie mit dem Betrag Null zugeführt wird, sodass die Amplitude der Phakonadel 5 den Betrug Null besitzt. Aufgrund des vollständigen Verzichtes an Energiezufuhr in diesem zweiten Zeitabschnitt ZA2 erfolgt eine maximal mögliche Abkühlung des Gewebes in der Umgebung der Phakonadel.

Nach dem zweiten Zeitabschnitt ZA2 folgt ein dritter Zeitabschnitt ZA3, der bei dieser Ausführungsform eine erste Zeitdauer ZD1, eine zweite Zeitdauer ZD2, eine dritte Zeitdauer ZD3 und eine vierte Zeitdauer ZD4 umfasst. In der ersten Zeitdauer ZD1 und in der dritten Zeitdauer ZD3 wird mittels der Steuerungsvorrichtung 1 elektrische Energie von der Energieversorgung 2 an den Aktuator 3 und somit an die Phakonadel 5 zugeführt, sodass Pulse mit einer konstanten Amplitude A2 anliegen. Die Amplitude A2 beträgt bei dieser Ausführungsform etwa 70 % der maximalen Amplitude A1 und ist damit niedriger als die maximale Amplitude A1 während des ersten Zeitabschnittes ZA1.

In der zweiten Zeitdauer ZD2 und in der vierten Zeitdauer ZD4 liegen Pulse mit einer konstanten Amplitude A3 an, die einen Betrag in Höhe von mehr als 0 % und weniger als 10 % der maximalen Amplitude A1 erreicht. Damit erfolgt in der zweiten Zeitdauer ZD2 und der vierten Zeitdauer ZD4 keine Emulsifikation der Augenlinse, sodass eine Abkühlung der Umgebung der Phakonadel stattfinden kann. Während des ersten Zeitabschnittes ZA1 und während des dritten Zeitabschnittes ZA3 erfolgt somit eine Bewegung der Phakonadel, wodurch jeweils der für den Aktuatorbetrieb erforderliche Strom und die abgefallene Spannung ermittelt werden kann. Dies ist vorteilhaft, da somit zum Beispiel eine Auswertung des Okklusionszustandes der Phakonadel in dieser Zeit möglich ist. Während des zweiten Zeitabschnittes ZA2 wird hingegen kein den Aktuatoren zugeführter Strom und auch keine an den Aktuatoren abgefallene Spannung ermittelt oder ein Okklusionszustand der Phakonadel erfasst. Der zweite Zeitabschnitt ZA2 dient ausschließlich dazu, eine maximale Abkühlung zu ermöglichen. Da die in dem zweiten Zeitabschnitt an den Aktuator zugeführte elektrische Energie einen Betrag gleich Null besitzt, wird auch kein Strom an die Aktuatoren zugeführt, sodass eine Auswertung des Stroms und einer an den Aktuatoren abgefallenen Spannung nicht erforderlich ist.

Nach dem dritten Zeitabschnitt ZA3 wiederholt sich der Pulsverlauf vom ersten Zeitabschnitt ZA1 bis zum dritten Zeitabschnitt ZA3, wodurch eine wirksame, aber im Hinblick auf eine Temperaturerhöhung im Bereich der Hornhaut sehr schonende Emulsifikation erreicht wird.

In Figur 3 ist ein Pulsverlauf gemäß einer zweiten Ausführungsform der Steuerungsvorrichtung dargestellt. Der erste Zeitabschnitt ZA1 und der zweite Zeitabschnitt ZA2 ist unverändert im Vergleich zu der Darstellung in Figur 1. Der dritte Zeitabschnitt ZA31 ist jedoch kürzer als der dritte Zeitabschnitt ZA3 gemäß Figur 2. Diese Verkürzung des dritten Zeitabschnittes wird durch eine entsprechende Stellung des Fußpedals 11 erreicht. Die Steuerungsvorrichtung kann so eingerichtet sein, dass sich bei zunehmendem Herunterdrücken des Fußpedals 11 der dritte Zeitabschnitt von der Länge ZA3 auf ZA31 zunehmend verkürzt. Damit kann zum Beispiel bei einer relativ harten Augenlinse etwas mehr Energie in die Augenlinse zugeführt werden.

Figur 4 zeigt eine schematische Darstellung eines Pulsverlaufes in Abhängigkeit von der Zeit gemäß einer dritten Ausführungsform der erfindungsgemäßen Steuerungsvorrichtung 1. Der Pulsverlauf ist mit dem ersten Zeitabschnitt ZA1 und dem zweiten Zeitabschnitt ZA2 identisch mit dem in Figur 1 dargestellten Pulsverlauf. Jedoch unterscheidet sich der Pulsverlauf des dritten Zeitabschnittes ZA32 vom dritten Zeitabschnitt ZA3 dadurch, dass die Pulse während anderer Zeitdauern ZD11, ZD21, ZD31 und ZD41 anliegen. Die Pulse während der zweiten Zeitdauer ZD21 und der vierten Zeitdauer ZD41 besitzen die gleiche Länge wie der Zeitabschnitt ZA2, in dem keine Pulse anliegen. Außerdem besitzen die Pulse während der dritten Zeitdauer ZD31 eine konstante Amplitude A4. Die Amplitude A4 ist niedriger als die Amplitude A1 und die Amplitude A2, jedoch höher als die Amplitude A3. Somit ergibt sich eine Stufung der Höhe der Amplituden von A1 auf A2 auf A4 auf A3, welches eine sehr sanfte Reduktion der zugeführten Schwingungsenergie in die Augenlinse bewirkt. Damit wird ein guter Kompromiss zwischen einer Emulsifikation in kurzer Zeit mit hohem Wirkungsgrad und einer geringen Gefahr für eine Verbrennung der Hornhaut aufgrund einer guten Abkühlung während des Emulsifikationsprozesses erreicht.

## Patentansprüche

1. Steuerungsvorrichtung für ein Phakoemulsifikationssystem, wobei die Steuerungsvorrichtung eingerichtet ist, einem Aktuator für eine Phakonadel während mehrerer Zeitabschnitte elektrische Energie zuzuführen, wobei die Zeitabschnitte umfassen:
- einen ersten Zeitabschnitt, in dem elektrische Energie für Pulse für eine Phakoemulsifikation zugeführt wird, wobei die Pulse eine konstante maximale Amplitude aufweisen,
- einen zweiten Zeitabschnitt, welcher sich dem ersten Zeitabschnitt anschließt, wobei in dem zweiten Zeitabschnitt elektrische Energie mit einem Betrag gleich Null zugeführt wird,
- einen dritten Zeitabschnitt, welcher sich dem zweiten Zeitabschnitt anschließt, wobei der dritte Zeitabschnitt eine erste Zeitdauer aufweist, in der elektrische Energie für Pulse für eine Phakoemulsifikation zugeführt wird, wobei die Pulse eine niedrigere konstante Amplitude als die maximale Amplitude während des ersten Zeitabschnittes aufweisen,
wobei auf die erste Zeitdauer eine zweite Zeitdauer folgt, in der Pulse mit einer Amplitude anliegen, die einen konstanten Betrag in Höhe von mehr als 0 % und weniger als 10 % der maximalen Amplitude erreicht, sodass während der zweiten Zeitdauer keine Phakoemulsifikation erfolgt,
wobei die erste Zeitdauer des dritten Zeitabschnittes kürzer ist als der erste Zeitabschnitt.

2. Steuerungsvorrichtung nach Anspruch 1, wobei die Länge des ersten Zeitabschnittes vorbestimmt ist und durch eine Verstellung eines Fußpedals nur die Länge des dritten Zeitabschnittes veränderbar ist.

3. Steuerungsvorrichtung nach Anspruch 1, wobei die Länge des dritten Zeitabschnittes vorbestimmt ist und durch eine Verstellung eines Fußpedals nur die Länge des ersten Zeitabschnittes veränderbar ist.

4. Steuerungsvorrichtung nach einem der vorherigen Ansprüche, wobei die Amplitude der Pulse der ersten Zeitdauer des dritten Zeitabschnittes in einem Bereich von 40 % bis 70 % der maximalen Amplitude des ersten Zeitabschnittes einstellbar ist.

5. Steuerungsvorrichtung nach einem der vorherigen Ansprüche, wobei der dritte Zeitabschnitt eine erste Zeitdauer und eine zweite Zeitdauer und direkt daran anschließend eine dritte Zeitdauer und eine vierte Zeitdauer aufweist, wobei in der dritten Zeitdauer elektrische Energie für Pulse zugeführt wird, welche eine niedrigere konstante Amplitude als während des ersten Zeitabschnittes aufweisen und in der vierten Zeitdauer Pulse mit einer Amplitude anliegen, die einen konstanten Betrag in Höhe von mehr als 0 % und weniger als 10 % der maximalen Amplitude erreicht.

6. Steuerungsvorrichtung nach einem der vorherigen Ansprüche, wobei durch eine Verstellung des Fußpedals das Verhältnis aus dem dritten Zeitabschnitt zu dem ersten Zeitabschnitt in einem Bereich von 0,5 bis 2, bevorzugt in einem Bereich von 0,5 bis 1, verstellbar ist.

7. Steuerungsvorrichtung nach einem der Ansprüche 5 oder 6, wobei die Pulse bei der dritten Zeitdauer eine niedrigere konstante Amplitude als bei der ersten Zeitdauer aufweisen.

8. Phakoemulsifikationssystem, welches eine Steuerungsvorrichtung nach einem der vorherigen Ansprüche aufweist.

## Claims

1. Control apparatus for a phacoemulsification system, wherein the control apparatus is configured to supply electrical energy to an actuator for a phaco needle during a plurality of time intervals, wherein the time intervals comprise:
- a first time interval, in which electrical energy for pulses for a phacoemulsification is supplied, wherein the pulses have a constant maximum amplitude,
- a second time interval following the first time interval, wherein electrical energy with a value equal to zero is supplied during the second time interval,
- a third time interval following the second time interval, wherein the third time interval has a first time duration in which electrical energy for pulses for a phacoemulsification is supplied, wherein the pulses have a lower constant amplitude that the maximum amplitude during the first time interval,
wherein the first time duration is followed by a second time duration in which pulses having an amplitude reaching a constant magnitude equal to more than 0% and less than 10% of the maximum amplitude are applied such that there is no phacoemulsification during the second time duration,
wherein the first time duration of the third time interval is shorter than the first time interval.

2. Control apparatus according to Claim 1, wherein the length of the first time interval is predetermined and only the length of the third time interval is modifiable by repositioning a foot pedal.

3. Control apparatus according to Claim 1, wherein the length of the third time interval is predetermined and only the length of the first time interval is modifiable by repositioning a foot pedal.

4. Control apparatus according to any one of the preceding claims, wherein the amplitude of the pulses in the first time duration of the third time interval is adjustable within a range of 40% to 70% of the maximum amplitude from the first time interval.

5. Control apparatus according to any one of the preceding claims, wherein the third time interval has a first time duration and a second time duration and, directly following this, a third time duration and a fourth time duration, wherein electrical energy for pulses which have a lower constant amplitude than during the first time interval is supplied during the third time duration and pulses with an amplitude reaching a constant magnitude equal to more than 0% and less than 10% of the maximum amplitude are applied during the fourth time duration.

6. Control apparatus according to any one of the preceding claims, wherein the ratio of the third time interval to the first time interval is adjustable in a range from 0.5 to 2, preferably in a range from 0.5 to 1, by repositioning the foot pedal.

7. Control apparatus according to either of Claims 5 and 6, wherein the pulses during the third time duration have a lower constant amplitude than during the first time duration.

8. Phacoemulsification system, having a control apparatus according to any one of the preceding claims.

## Revendications

1. Dispositif de commande pour un système de phacoémulsification, le dispositif de commande étant conçu pour acheminer de l'énergie électrique à un actionneur pour une aiguille de phacoémulsification pendant plusieurs intervalles de temps, les intervalles de temps comprenant :
- un premier intervalle de temps dans lequel est acheminé de l'énergie électrique pour les impulsions destinées à une phacoémulsification,
- un deuxième intervalle de temps qui se rattache au premier intervalle de temps, de l'énergie électrique ayant une valeur égale à zéro étant acheminée dans le deuxième intervalle de temps,
- un troisième intervalle de temps qui se rattache au deuxième intervalle de temps, le troisième intervalle de temps possédant une première durée dans laquelle est acheminée de l'énergie électrique pour les impulsions destinées à une phacoémulsification, les impulsions possédant une amplitude constante plus faible que l'amplitude maximale pendant le premier intervalle de temps,
la première durée étant suivie par une deuxième durée dans laquelle sont appliquées des impulsions ayant une amplitude qui atteint une valeur constante à hauteur de plus de 0 % et de moins de 10 % de l'amplitude maximale, de sorte qu'une phacoémulsification n'a pas lieu pendant la deuxième durée,
la première durée du troisième intervalle de temps étant plus courte que le premier intervalle de temps.

2. Dispositif de commande selon la revendication 1, la longueur du premier intervalle de temps étant prédéterminée et seule la longueur du troisième intervalle de temps pouvant être modifiée par un positionnement d'une pédale.

3. Dispositif de commande selon la revendication 1, la longueur du troisième intervalle de temps étant prédéterminée et seule la longueur du premier intervalle de temps pouvant être modifiée par un positionnement d'une pédale.

4. Dispositif de commande selon l'une des revendications précédentes, l'amplitude des impulsions de la première durée du troisième intervalle de temps étant réglable dans une plage de 40 % à 70 % de l'amplitude maximale du premier intervalle de temps.

5. Dispositif de commande selon l'une des revendications précédentes, le troisième intervalle de temps possédant une première durée et une deuxième durée et, directement rattachées à celle-ci, une troisième durée et une quatrième durée, de l'énergie étant acheminée dans la troisième durée pour des impulsions qui possèdent une amplitude constante plus faible que pendant le premier intervalle de temps et des impulsions étant appliquées dans la quatrième durée avec une amplitude qui atteint une valeur constante à hauteur de plus de 0 % et de moins de 10 % de l'amplitude maximale.

6. Dispositif de commande selon l'une des revendications précédentes, le rapport entre le troisième intervalle de temps et le premier intervalle de temps pouvant être réglé dans une plage de 0,5 à 2, de préférence dans une plage de 0,5 à 1, par un positionnement de la pédale.

7. Dispositif de commande selon l'une des revendications 5 ou 6, les impulsions lors de la troisième durée possédant une amplitude constante plus faible que lors de la première durée.

8. Système de phacoémulsification qui possède un dispositif de commande selon l'une des revendications précédentes.
